# EUROPEAN PATENT APPLICATION

(11) **EP 4 616 900 A1**
(43) Date of publication of application: **17.09.2025**
(21) Application number: 23907220.0
(22) Date of filing: 17.01.2023
(51) Int. Cl.: A61N 2/02, A61N 2/00, H01F 27/28

(54) **HELMET-TYPE MAGNETIC STIMULATION DEVICE**

(30) Priority: 19.12.2022 KR 20220178265
(71) Applicant: Remed Brainstim Co., Ltd., Seongnam-si Gyeonggi-do 13647 (KR)
(72) Inventor: YOON, Se Jin, Anyang-si, Gyeonggi-do 14055 (KR); KIM, Il Kyu, Suwon-si, Gyeonggi-do 16354 (KR); KIM, Young Kwang, Seoul 01318 (KR); CHO, Sung Taek, Seoul 07786 (KR)
(74) Representative: ABG Intellectual Property Law, S.L.
(86) International application number: PCT/KR2023/000787
(87) International publication number: WO 2024/135925

(57) **Abstract**

The present invention relates to a helmet-type magnetic stimulation device that provides non-invasive treatment by applying a magnetic field to cranial nerve cells or scalp cells, the helmet-shaped magnetic stimulation device comprising: a helmet-shaped case part comprising a lower case part which is disposed spaced apart by a predetermined distance from the upper part of the skull and an upper case part which is coupled to the top of the lower case part and forms an inner space; a coil-fixing part installed in the lower case part; and a magnetism-generation coil seated on the coil-fixing part and the generating a magnetic field.

## Description

### TECHNICAL FIELD

The present invention relates to a helmet-type magnetic stimulation device, and more particularly, to a helmet-type magnetic stimulation device that performs non-invasive treatment by applying a magnetic field to a scalp or cranial nerves of a human body.

### BACKGROUND ART

Transcranial magnetic stimulation (TMS) is a brain stimulation technique that uses local magnetic field waves induced on a surface of a head outside a human body to activate or suppress nerve cells in specific areas of a brain. The principle of the TMS is to apply strong electric current to an electromagnetic coil, thereby generating a magnetic field of a degree of several Tesla. This is a method for stimulating the brain by transmitting fluctuating energy of the magnetic field waves to the brain and inducing depolarization in nerve cells within a range of several centimeters below the coil.

The TMS is a representative non-invasive brain stimulation technique, and its therapeutic application is being actively studied in depression, insomnia, obsessive-compulsive disorder, movement disorders, dementia, and brain dysfunction, which do not respond well to drug treatment and psychotherapy.

Typically, the recommended treatment time for the TMS is 5 days a week, once a day, for about 20 minutes, and thus, frequent treatments are required. For example, when magnetic stimulation therapy is applied repeatedly for about 20 minutes to about 50 minutes a day for several days to central nervous system diseases such as stroke or spinal cord injury, effects of neuroplasticity or nerve regeneration and functional recovery have been reported.

However, since existing transcranial magnetic stimulation devices are non-movable, it is difficult to match this treatment cycle to the domestic medical market situation. Thus, there is a need to introduce a miniaturized TMS treatment device that is capable of being easily used at home.

Alopecia refers to a condition in which there are fewer hairs in an area on which there should normally be two. It is known that this type of alopecia is caused by various factors such as genetic factors, male hormones, stress, and environmental pollution. Recently, as the number of young people in their 20s and 30s and women with alopecia increases, it has become a serious social problem, and interest in alopecia treatment devices is also increasing.

Recently, a helmet-type treatment device that installs laser diodes on protrusions of the treatment device to generate wavelengths that are optimal for scalp growth is being spotlighted as the alopecia treatment device used at home. This helmet-type alopecia treatment device irradiates laser light to the scalp to allow the light energy to pass through a dermal tissue and subcutaneous tissue, thereby activating various cell tissues including hair roots.

However, these conventional technologies have the problem that their therapeutic effects are not high because of simply relying on light therapy using lasers, and the technologies attempt to promote hair growth and prevent alopecia by activating the scalp's biological tissues through a negative pressure by adding vibration therapy or ultrasonic therapy. However, there is a problem that an effect of the method of activating the scalp's tissues through a method for combining the vibration or ultrasonic waves is not high because the combined effect of the light and vibration or the ultrasonic waves is not great.

### DISCLOSURE OF THE INVENTION

### TECHNICAL PROBLEM

The present invention has been made to solve the above-mentioned conventional problems, and an object of the present invention is to provide a helmet-type magnetic stimulation device provided with a magnetism-generation coil that generates a magnetic field to apply the magnetic field to scalp or cranial nerves and a cooling part that cools heat generated from the magnetism-generation coil.

In addition, an object of the present invention is to provide a structure in which a lower case part and an upper case part, which are provided as a case part, are detachably coupled to each other and which includes a coil-fixing part installed in the lower case part to stably support the magnetism-generation coil in an internal space of the case part.

In addition, an object of the present invention is to provide a structure in which a blowing device and a cooling hole, which are provided as a cooling part, are disposed symmetrically in a casing to facilitate circulation of external air, thereby optimizing cooling efficiency for cooling heat of the magnetism-generation coil.

### TECHNICAL SOLUTION

A helmet-type magnetic stimulation device that provides non-invasive treatment by applying a magnetic field to cranial nerve cells or scalp cells according to the present invention for achieving the above objects includes: a helmet-shaped case part comprising a lower case part disposed to be spaced a predetermined distance from an upper portion of the skull and an upper case part coupled to an upper portion of the lower case part to define an internal space; a coil-fixing part installed in the lower case part; and a magnetism-generation coil seated on the coil-fixing part to generate the magnetic field, wherein the coil-fixing part includes: a first support part configured to surround an outer circumference of the magnetism-generation coil; and a second support part configured to surround an inner circumference of the magnetism-generation coil.

In addition, in the helmet-type magnetic stimulation device, the coil-fixing part may further include a third support part disposed between the first support part and the second support part to support a bottom surface of the coil so that the coil is spaced a predetermined distance from the lower case part.

In addition, in the helmet-type magnetic stimulation device, the helmet-type magnetic stimulation device may further include a cooling part configured to generate an air flow in the internal space so as to cool the magnetism-generation coil, wherein the cooling part may include: a blowing device installed in the upper case part to generate the air flow in the internal space; and a cooling hole defined in the lower case part and opened to allow air to be introduced into the internal space or to be discharged from the internal space, wherein the cooling hole may be defined in an area between the first support part and the second support part.

In addition, in the helmet-type magnetic stimulation device, the cooling hole may be a hole defined to pass from a lower side of the magnetism-generation coil to the lower case part.

In addition, in the helmet-type magnetic stimulation device, the cooling hole may be defined adjacent to the second support part.

In addition, in the helmet-type magnetic stimulation device, the cooling part may further include a fluid guide part configured to guide a fluid introduced or discharged through the cooling hole, wherein the fluid guide part may be a rib member extending from the cooling hole and installed in the lower case part.

In addition, the helmet-type magnetic stimulation device may further include a coil cover member configured to cover at least a portion of the magnetism-generation coil.

In addition, in the helmet-type magnetic stimulation device, a fluid guide protrusion may be disposed on an inner surface of the coil cover member facing the magnetism-generation coil.

### ADVANTAGEOUS EFFECTS

As described above, the present invention may provide the helmet-shaped magnetic stimulation device provided with the magnetic field generation part, which generates the magnetic field to apply the magnetic field to the scalp or cranial nerves, and the cooling part, which cools the heat generated from the magnetic field generation part, to enable the user to perform the scalp or cranial stimulation without the time and space restriction.

In addition, according to the helmet-type magnetic stimulation device of the present invention, the coil-fixing part is installed in the lower case part to stably support the magnetism-generation coil in the internal space of the case part.

In addition, according to the helmet-type magnetic stimulation device of the present invention, the blowing device and the cooling hole may be disposed symmetrically in the casing to facilitate the circulation of the external air, thereby improving the cooling efficiency for cooling the heat of the magnetism-generation coil.

In addition, according to the helmet-type magnetic stimulation device of the present invention, the coil cover member that covers the portion of the magnetism-generation coil and the fluid guide protrusion disposed inside the coil cover member may be provided so that the fluid introduced from the outside stays around the magnetism-generation coil for the predetermined time and then is discharged, thereby improving the cooling efficiency.

### BRIEF DESCRIPTION OF THE DRAWINGS

FIG. 1 is a perspective view of a helmet-type magnetic stimulation device according to an embodiment of the present invention.
FIG. 2 is an exploded view illustrating a case part of the helmet-type magnetic stimulation device according to an embodiment of the present invention.
FIG. 3 is an exploded perspective view of the helmet-type magnetic stimulation device according to an embodiment of the present invention.
FIG. 4 is a view illustrating a configuration of the helmet-type magnetic stimulation device when viewed from bottom according to an embodiment of the present invention.
FIG. 5 is an embodied view illustrating a coil-fixing part of the helmet-type magnetic stimulation device according to an embodiment of the present invention.
FIG. 6 is an embodied view illustrating a coil cover member of the helmet-type magnetic stimulation device according to an embodiment of the present invention.
FIG. 7 is a view illustrating various modified structures of a coil-fixing part of a helmet-type magnetic stimulation device according to another embodiment of the present invention.

### MODE FOR CARRYING OUT THE INVENTION

Advantages and features of the present disclosure, and implementation methods thereof will be clarified through following embodiments described with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art. Further, the present invention is only defined by scopes of claims.

Although the terms first, second, etc. are used to describe various components, these components are not limited by these terms. These terms are only used to distinguish one component from another component. Accordingly, a first component that will be described below may be a second component within the technical idea of the present disclosure.

In the embodiments below, the terms such as "include" or "have" mean that a feature or component described in the specification is present, and do not exclude in advance the possibility that one or more other features or components may be added.

In the drawings, for convenience of description, the dimensions of elements are exaggerated or downscaled. For example, since the size and shape of each of the components illustrated in the drawings are arbitrarily shown for convenience of description, the present invention is not necessarily limited to the illustrated.

Like reference numerals refer to like elements throughout.

Each feature of the various embodiments of the present invention can be partially or fully coupled or combined with each other, and as can be fully understood by those skilled in the art, various technical interconnections and operations are possible. Also, the embodiments may be independently performed with respect to each other or performed in combination of each other.

Hereinafter, a helmet-type magnetic stimulation device 100 of the present invention will be described in detail with reference to the attached drawings.

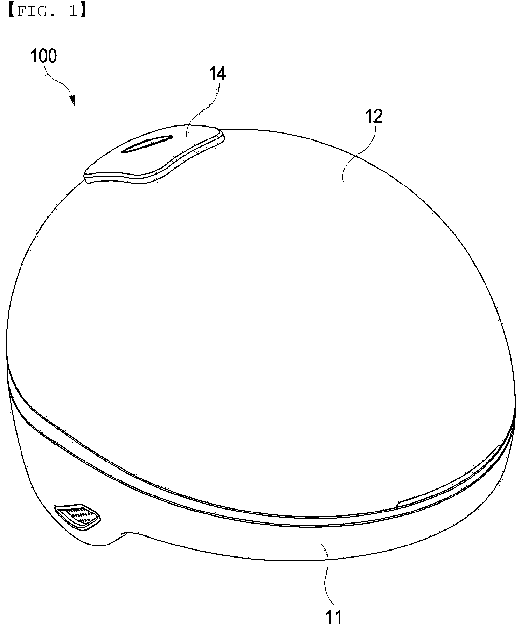

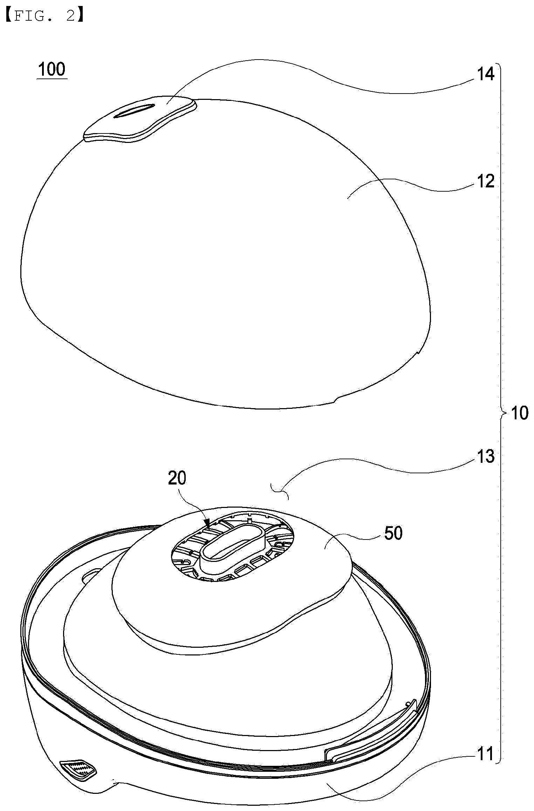

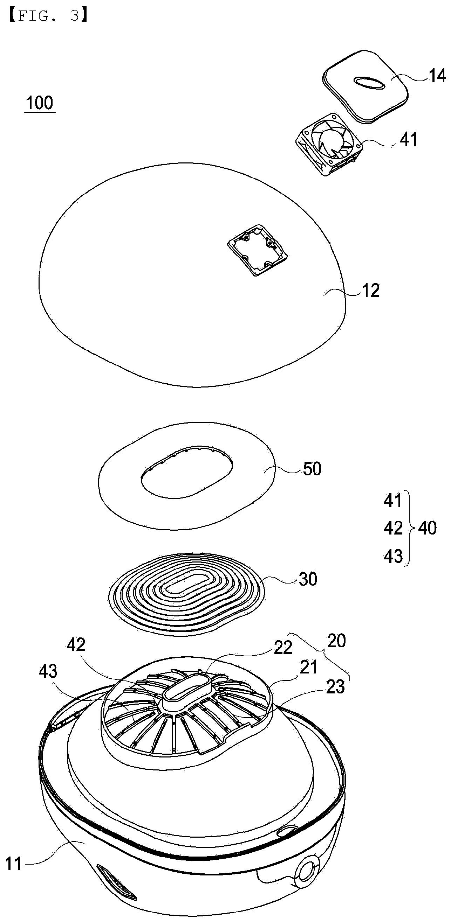

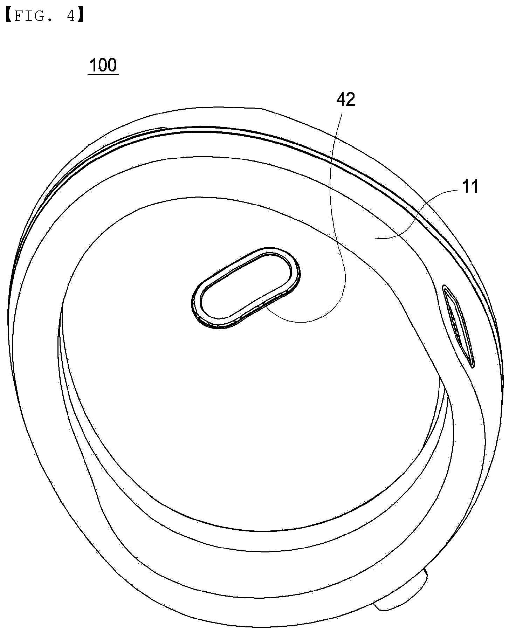

FIG. 1 is a perspective view of a helmet-type magnetic stimulation device according to an embodiment of the present invention, FIG. 2 is an exploded view illustrating a case part of the helmet-type magnetic stimulation device according to an embodiment of the present invention, FIG. 3 is an exploded perspective view of the helmet-type magnetic stimulation device according to an embodiment of the present invention, and FIG. 4 is a view illustrating a configuration of the helmet-type magnetic stimulation device when viewed from bottom according to an embodiment of the present invention.

The helmet-type magnetic stimulation device 100 of the present invention provides non-invasive treatment by applying a magnetic field to intracranial nerve cells or scalp cells. As illustrated in FIGS. 1 to 3, the helmet-type magnetic stimulation device 100 according to an embodiment of the present invention includes a case part 10, a coil-fixing part 20, a magnetism-generation coil 30, a cooling part 40, and a coil cover member 50.

The case part 10 is a helmet-shaped case member mounted on an upper portion of a skull of the human body and includes a lower case part 11 disposed to be spaced a predetermined distance from the upper portion of the skull, and an upper case part 12 coupled to the upper portion of the lower case part 11 to define an internal space 13.

The lower case part 11 and the upper case part 12 are formed to be convex upward so as to be in contact with the skull of the human head and may be mounted to be spaced a predetermined distance from the human scalp. The lower case part 11 and the upper case part 12 form the internal space 13 in which the magnetism-generation coil 30 is installed and a cooling space through the cooling part 40.

The case part 10 may further include a blowing device cover 14 mounted on a blowing device 41 described later, and the blowing device cover 14 may be coupled to the upper case part 12 or the blowing device 41 as illustrated in FIG. 3.

The coil fixing member 20 is a member that supports or fixes the magnetism-generation coil 30 and is disposed on the lower case part 11 as illustrated in FIG. 3. The coil-fixing part 20 may be provided to be integrated with the lower case part 11 or may be provided as a separate member and fixed to be seated on the lower case part 11.

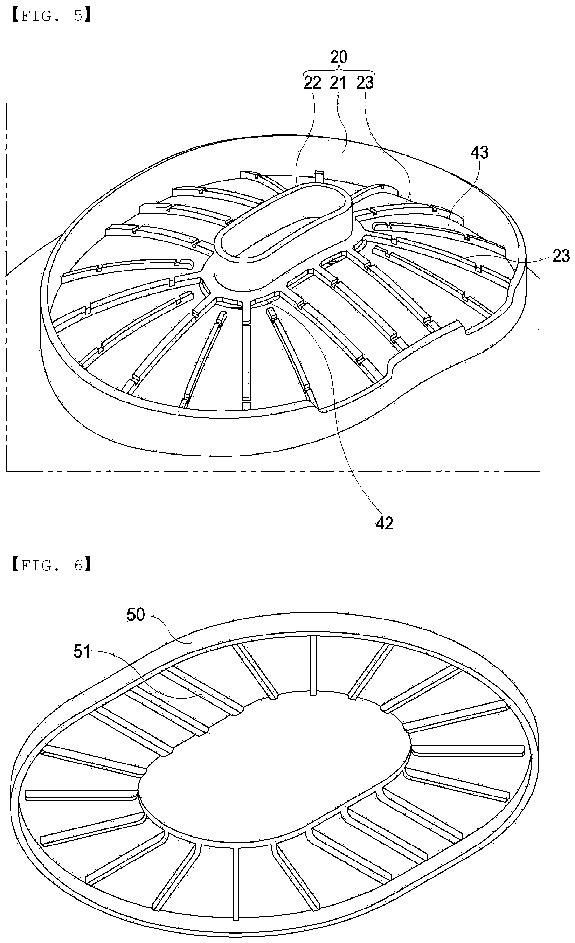

FIG. 5 is an embodied view illustrating the coil-fixing part 20 of the helmet-type magnetic stimulation device 100 according to an embodiment of the present invention.

The coil fixing member 20 includes a first support member 21 disposed to support an outer circumference of the magnetism-generation coil 30 and a second support part 22 that supports an inner circumference of the magnetism-generation coil 30 when the magnetism-generation coil 30 is seated on the coil fixing member 20.

The first support member 21 and the second support member 22 are configured to support the magnetism-generation coil 30 and serve to prevent the magnetism-generation coil 30 from moving in a direction parallel to the lower case part 11. It is preferable that the first support member 21 and the second support member 22 be provided at a height corresponding to a height of the magnetism-generation coil 30 or a coil diameter of the magnetism-generation coil 30.

As illustrated in FIG. 5, a bent part may be disposed so that a user easily inserts and withdraws the magnetism-generation coil 30 into/from the coil fixing member 20.

In addition, the coil fixing member 20 may further include a third support member 23 disposed between the first support member 21 and the second support member 22 to support a bottom surface of the magnetism-generation coil 30. The third support member 23 allows the magnetism-generation coil 30 to be spaced a predetermined distance from the lower case part 11. The third support member 23 serves to define a space having a predetermined interval at a lower side of the magnetism-generation coil 30 so that the fluid that cools the magnetism-generation coil 30 is introduced into or discharged from the cooling hole 42 described later.

The third support member 23 may be a plurality of radial rib members connecting the first annular support member 21 to the second annular support member 22, as illustrated in FIG. 5. However, the third support member 23 may not be limited to the illustrated shape and may have a different shape as long as the third support member 23 supports the installed magnetism-generation coil 30.

The magnetism-generation coil 30 is a member seated on the coil-fixing part 20 to generate a magnetic field, i.e., a coil wound to have a predetermined diameter. The magnetic field generated from the magnetism-generation coil 30 may be adjusted in output to treat various indications such as depression, autism, dementia, insomnia, and alopecia.

In the helmet-type magnetic stimulation device according to an embodiment of the present invention, the magnetism-generation coil 30 is exemplified as having an overall elliptical shape, but is not limited thereto. The magnetism-generation coil 30 may be a coil wound to have various shapes such as a circular, elliptical, figure-8, disk, polygonal or butterfly shape, and it should be understood that the magnetism-generation coil 30 encompasses the general shapes of the conventional TMS coils.

The cooling part 40 generates a flow of the fluid, i.e. air, in the internal space of the case to cool the magnetism-generation coil 30. The cooling part 40 includes a blowing device 41 that blows air to the magnetism-generation coil 30, a cooling hole 42, and a fluid guide part 43.

The blowing device 41 is installed in the upper case part 12 to generates the air flow in the internal space 13 of the case part 10. The blowing device 41 is a device that blows air into the internal space 13 to cool the heat generated from the magnetic field generation part 30 and includes a cooling means such as a fan or blower.

The blowing device 41 is installed at a front or rear side of the upper case part 12 to introduce or discharge the air into/from the internal space of the case part, thereby generating the air flow in the internal space 13.

The cooling hole 42 is an opened hole defined in the lower case part 11 to allow the air to be introduced into the internal space 13 or to be discharged from the internal space. FIG. 4 illustrates a configuration in which the cooling hole 42 is defined to pass through the lower case part 11.

It is preferable that the cooling hole 42 is defined in an area between the first support part 21 and the second support part 22 of the coil-fixing part 20. In addition, it is preferable that the cooling hole 42 is defined at a lower side of the magnetism-generation coil 30. In addition, the cooling hole 42 may be defined adjacent to the second support member 22 that supports the inner circumference of the magnetism-generation coil 30, as illustrated in FIG. 5.

The cooling hole 42 may be defined as an annular hole along a circumference of the annular second support member 22 as illustrated in the drawings or may be defined as a hole having any shape that is spaced at a predetermined interval.

As illustrated in FIG. 5, when the cooling hole 42 is defined below the magnetism-generation coil 30 to be adjacent to the second support part 22 that supports the inner circumference of the magnetism-generation coil 30, particularly, on the area between the first support part 21 and the second support part 22, the external air may be introduced from a central lower end of the coil to cool the entire bottom surface of the magnetism-generation coil 30 and then be discharged to the outside through the blowing device 41. The configuration and arrangement of the cooling part 40 have advantageous in that the cooling part promotes uniform cooling of the magnetism-generation coil 30 that is a heating element.

In addition, the cooling part 40 may further include the fluid guide part 43 that guides the fluid introduced or discharged through the cooling hole 42. The fluid guide part 43 may be a rib member that extends from the cooling hole 42 to guide the fluid. The fluid guide part 43 defines an area through which the air introduced into or discharged from the cooling hole 42 mainly passes.

The fluid guide part 43 may be a rib member installed in the lower case member 12, as illustrated in FIG. 5. The fluid guide part 43 may be provided in a shape similar to that of the third support part 23 that supports the bottom surface of the magnetism-generation coil 30. The fluid guide part 43 may be a plurality of radial rib members disposed between the first annular support member 21 and the second annular support member 22 and may be arranged alternately with the third support member 23. However, the fluid guide part 43 is not limited to the illustrated shape, and may have a shape different from the illustrated shape as long as the fluid guide part 43 has a shape so that the fluid introduced from the cooling hole 42 is uniformly spread around the magnetism-generation coil 30.

The cooling part 40 described above, particularly the cooling hole 42 and the fluid guide part 43 have technical features for maximizing the cooling performance of the helmet-type magnetic stimulation device 100 of the present invention. The bottom surface of the magnetism-generation coil 30 is a portion which is in close contact with the human body and in which the greatest amount of heat is generated. In particular, in the case of a cooling fluid circulation in which the fluid introduced through the cooling hole 42 is discharged from the blowing device 41, the cooling hole 42 and the fluid guide part 43 are essential components that allow external air having a low temperature to be first in contact with the bottom surface of the magnetism-generation coil 30, and thus, the cooling efficiency in this portion may be greatly improved.

The helmet-type magnetic stimulation device 100 according to an embodiment of the present invention may include the coil cover member 50. FIG. 6 is an embodied view illustrating the coil cover member 50 of the helmet-type magnetic stimulation device 100 according to an embodiment of the present invention.

The coil cover member 50 is a member that covers at least a portion of the magnetism-generation coil 30.

The coil cover member 50 is provided in a structure that partially covers a top surface of the magnetism-generation coil 30 when the magnetism-generation coil 30 is seated on the coil fixing member 20, to allow the magnetism-generation coil 30 to be stably supported on the helmet-type magnetic stimulation device 100 of the present invention. In addition, in the case of a cooling fluid circulation in which the fluid introduced through the cooling hole 42 is discharged from the blowing device 41, the coil cover member 50 may improve the cooling efficiency of the helmet-type magnetic stimulation device 100 of the present invention by allowing the fluid introduced through the cooling hole 42 to stay around the magnetism-generation coil 30 for a predetermined time and then move.

The coil cover member 50 may be made of, for example, an elastic rubber material. In addition, a fluid guide protrusion 51 may be disposed on an inner surface of the coil cover member 50. FIG. 6 illustrates a configuration when viewed from the inner surface of the coil cover member 50 facing the magnetism-generation coil 30.

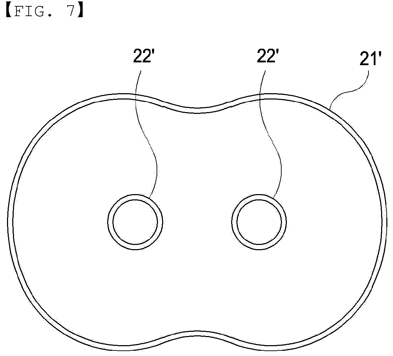

FIG. 7 is a view illustrating various modified structures of a coil-fixing part of a helmet-type magnetic stimulation device according to another embodiment of the present invention.

As described above, a magnetism-generation coil 30 may be a coil wound to have various shapes such as a circular, figure-8, or butterfly shape in addition to the oval shape illustrated in FIGS. 1 to 6.

FIG. 7 illustrates an example of a first support part 21' and a second support part 22' of a coil-fixing part capable of supporting the figure-8 shaped coil when the magnetism-generation coil 30 has the figure-8 shape. The first support part 21' is a support part that supports an outer circumference of the figure-8 coil, and the second support part 22' is a support part that supports two inner circumferences of the figure-8 coil. It is made clear that such modified forms may also be included in the scope of the present invention.

Although the embodiment of the inventive concept is described with reference to the accompanying drawings, those with ordinary skill in the technical field of the inventive concept pertains will be understood that the present disclosure can be carried out in other specific forms without changing the technical idea or essential features. Therefore, the above-disclosed embodiments are to be considered illustrative and not restrictive.

## Claims

1. A helmet-type magnetic stimulation device that provides non-invasive treatment by applying a magnetic field to cranial nerve cells or scalp cells, the helmet-shaped magnetic stimulation device comprising:
a helmet-shaped case part comprising a lower case part disposed to be spaced a predetermined distance from an upper portion of the skull and an upper case part coupled to an upper portion of the lower case part to define an internal space;
a coil-fixing part installed in the lower case part; and
a magnetism-generation coil seated on the coil-fixing part to generate the magnetic field,
wherein the coil-fixing part comprises:
a first support part configured to surround an outer circumference of the magnetism-generation coil; and
a second support part configured to surround an inner circumference of the magnetism-generation coil.

2. The helmet-type magnetic stimulation device of claim 1, wherein the coil-fixing part further comprises a third support part disposed between the first support part and the second support part to support a bottom surface of the coil so that the coil is spaced a predetermined distance from the lower case part.

3. The helmet-type magnetic stimulation device of claim 1, further comprising a cooling part configured to generate an air flow in the internal space so as to cool the magnetism-generation coil,
wherein the cooling part comprises:
a blowing device installed in the upper case part to generate the air flow in the internal space; and
a cooling hole defined in the lower case part and opened to allow air to be introduced into the internal space or to be discharged from the internal space,
wherein the cooling hole is defined in an area between the first support part and the second support part.

4. The helmet-type magnetic stimulation device of claim 3, wherein the cooling hole is a hole defined to pass from a lower side of the magnetism-generation coil to the lower case part.

5. The helmet-type magnetic stimulation device of claim 3, wherein the cooling hole is defined adjacent to the second support part.

6. The helmet-type magnetic stimulation device of claim 3, wherein the cooling part further comprises a fluid guide part configured to guide a fluid introduced or discharged through the cooling hole,
wherein the fluid guide part is a rib member extending from the cooling hole and installed in the lower case part.

7. The helmet-type magnetic stimulation device of claim 1, further comprising a coil cover member configured to cover at least a portion of the magnetism-generation coil.

8. The helmet-type magnetic stimulation device of claim 7, wherein a fluid guide protrusion is disposed on an inner surface of the coil cover member facing the magnetism-generation coil.
